# EUROPEAN PATENT APPLICATION

(11) **EP 3 679 993 A1**
(43) Date of publication of application: **15.07.2020**
(21) Application number: 19151158.3
(22) Date of filing: 10.01.2019
(51) Int. Cl.: A63B 69/36, A63B 43/06

(54) **SENSING MEANS**

(71) Applicant: Nokia Technologies Oy, 02610 Espoo (FI)
(72) Inventor: MÜLLER, Kiti, 00430 Helsinki (FI); LINDHOLM, Harri, 00320 Helsinki (FI); KOLMONEN, Veli-Matti, 02610 Espoo (FI); PELLIKKA, Jarkko, 00220 Helsinki (FI)
(74) Representative: Whiting, Gary

(57) **Abstract**

An apparatus, method and computer program are described, comprising: providing a prompt to a user of a sensing ball; generating data relating to an interaction of a foot of the user with the sensing ball in response to the prompt; and processing said data relating to the interaction of the foot of the user with the sensing ball.

## Description

### Field

The present specification relates to a sensing means for receiving or generating data relating to an interaction of a foot of a user with the sensing means.

### Background

Feet and ankles are complex structures of bones, joints, muscles and sensory and motor nerves. It is difficult to model the versatile dynamics of the leg. There remains a need for a sensing means for gathering data regarding the interaction of the foot of a user with the sensing means.

### Summary

In a first aspect, there is provided an apparatus (e.g. a control module, for example of a sensing ball) comprising: an actuator means for providing a prompt to a user of a sensing ball; a sensing means for generating data relating to an interaction of a foot of the user with the sensing ball in response to the prompt; and a processing means for processing said data (e.g. by one or more of gathering, storing and manipulating said data) relating to the interaction of the foot of the user with the sensing ball. Data relating to the interaction of the foot of the user with the sensing ball may be combined with other metrics (e.g. one or more of cardiovascular measurements, breathing, eye movement, electroencephalogram (EEG) data etc.). The sensing ball may be compressible such that it can react to one or more forces applied by the foot of a user. The sensing ball may, for example, comprise a fluid cavity.

The sensing ball may have a generally spherical shape, but this is not a requirement of all embodiments. For example, the sensing ball may deviate slightly from a perfectly spherical shape. Moreover, the sensing balls maybe non-spherical, such as other shapes that can be manipulated in similar ways to balls (such as tubes).

The data generated by the sensing means may take many forms, including one or more of: changes in pressure, friction, and motion induced on the sensing ball by the action of the user's foot.

The actuator means may comprise a user interface, such as an external user interface. The user interface may provide instructions to the user regarding how to interact with the sensing ball.

The actuator means may comprises one or more of: a haptic actuator; a vibration means; one or more protrusions having controllable dimensions; light; sound generating means; and heating and/or cooling means.

The sensing means may comprise a plurality of sensors. The plurality of sensors may comprise one or more of: one or more gyroscopes (e.g. to measure rotation of the sensing ball); one or more accelerometers; one or more inertial magnetometers; one or more force sensors (e.g. to measure pressing on the sensing ball by a foot of the user); and one or more pressure sensors (e.g. to measure pressing on the sensing ball by a foot of the user).

The processing means may comprise means for storing data, such as a data-logger.

The processing means may comprise a communication means for communicating with a control module. The communication means may, for example, comprise a wired or wireless communication means. The communication means may be configured to provide processed data to the control module. Alternatively, or in addition, the communication means may be configured to receive instructions (e.g. prompt instructions) from the control module.

The processing means may comprise a timer for recording a relationship between the prompt to said user and the interaction of the foot of the user with the sensing ball.

In a second aspect, this specification describes a sensing ball comprising an apparatus as described with reference to the first aspect. The sensing ball may be compressible. Alternatively, or in addition, the sensing ball may comprise a fluid cavity. The sensing ball may have a generally spherical shape, but this is not a requirement of all embodiments. For example, the sensing ball may deviate slightly from a perfectly spherical shape. Moreover, the sensing balls maybe non-spherical, such as other shapes that can be manipulated in similar ways to balls (such as tubes).

In a third aspect, this specification describes a method comprising: providing a prompt to a user of a sensing ball; generating data relating to an interaction of a foot of the user with the sensing ball in response to the prompt; and processing said data (e.g. by one or more of gathering, storing and manipulating said data) relating to the interaction of the foot of the user with the sensing ball. Data relating to the interaction of the foot of the user with the sensing ball may be combined with other metrics (e.g. one or more of cardiovascular measurements, breathing, eye movement, electroencephalogram (EEG) data etc.)

The sensing ball may have a generally spherical shape, but this is not a requirement of all embodiments. For example, the sensing ball may deviate slightly from a perfectly spherical shape. Moreover, the sensing balls maybe non-spherical, such as other shapes that can be manipulated in similar ways to balls (such as tubes).

The generated data may take many forms, including one or more of: changes in pressure, friction, and motion induced on the sensing ball by the action of the user's foot.

The prompt may be provided to the user of the sensing ball by a user interface, such as an external user interface. The user interface may provide instructions to the user regarding how to interact with the sensing ball.

The prompt may be provided to the user by one or more of: a haptic actuator; a vibration means; one or more protrusions having controllable dimensions; light; sound generating means; and heating and/or cooling means.

In some embodiments, the generated data is stored, for example, at a data-logger.

In some embodiments, processed data is communicated (e.g. wirelessly transmitted) to a control module. Alternatively, or in addition, instructions (e.g. prompt instructions) may be received from the control module.

In a fourth aspect, this specification describes any apparatus configured to perform any method as described with reference to the third aspect.

In a fifth aspect, this specification describes computer-readable instructions which, when executed by computing apparatus, cause the computing apparatus to perform any method as described with reference to the third aspect.

In a sixth aspect, this specification describes a computer program comprising instructions for causing an apparatus to perform at least the following: receive a prompt to a user of a sensing ball; generate data relating to an interaction of a foot of the user with the sensing ball in response to the prompt; and process said data (e.g. by one or more of gathering, storing and manipulating said data) relating to the interaction of the foot of the user with the sensing ball. Data relating to the interaction of the foot of the user with the sensing ball may be combined with other metrics (e.g. one or more of cardiovascular measurements, breathing, eye movement, electroencephalogram (EEG) data etc.).

In a seventh aspect, this specification describes a computer-readable medium (such as a non-transitory computer readable medium) comprising program instructions stored thereon for performing at least the following: providing a prompt to a user of a sensing ball; generating data relating to an interaction of a foot of the user with the sensing ball in response to the prompt; and processing said data (e.g. by one or more of gathering, storing and manipulating said data) relating to the interaction of the foot of the user with the sensing ball. Data relating to the interaction of the foot of the user with the sensing ball may be combined with other metrics (e.g. one or more of cardiovascular measurements, breathing, eye movement, electroencephalogram (EEG) data etc.).

In an eighth aspect, this specification describes an apparatus comprising: at least one processor; and at least one memory including computer program code which, when executed by the at least one processor, causes the apparatus to: receive a prompt to a user of a sensing ball; generate data relating to an interaction of a foot of the user with the sensing ball in response to the prompt; and process said data (e.g. by one or more of gathering, storing and manipulating said data) relating to the interaction of the foot of the user with the sensing ball. Data relating to the interaction of the foot of the user with the sensing ball may be combined with other metrics (e.g. one or more of cardiovascular measurements, breathing, eye movement, electroencephalogram (EEG) data etc.).

In a ninth aspect, this specification describes an apparatus (such as a control module or a server) comprising: a first input for receiving a prompt to a user of a sensing ball; a first control module for generating data relating to an interaction of a foot of the user with the sensing ball in response to the prompt; and a second control module for processing said data (e.g. by one or more of gathering, storing and manipulating said data) relating to the interaction of the foot of the user with the sensing ball. The first and second control modules may be implemented by the same control module. As noted above, the sensing ball may have a generally spherical shape, but this is not a requirement of all embodiments.

### Brief description of the drawings

Example embodiments will now be described, by way of non-limiting examples, with reference to the following schematic drawings, in which:
FIGS. 1 to 4 are schematic views showing example uses of a sensing ball in accordance with example embodiments;
FIG. 5 is a block diagram of a system in accordance with an example embodiment;
FIG. 6 shows a portion of a sensing ball in accordance with an example embodiment;
FIG. 7 is a block diagram of a system in accordance with an example embodiment;
FIG. 8 is a flow chart showing an algorithm in accordance with an example embodiment;
FIGS. 9 to 11 are systems demonstrating example uses of an apparatus in accordance with example embodiments;
FIG. 12 shows a sensing ball demonstrating principles in accordance with an example embodiment;
FIG. 13 shows a sensing ball demonstrating principles in accordance with an example embodiment;
FIG. 14 is a block diagram of components of a system in accordance with an example embodiment; and
FIGS. 15A and 15B show tangible media, respectively a removable memory unit and a compact disc (CD) storing computer-readable code which when run by a computer perform operations according to example embodiments.

### Detailed description

Feet and ankles are complex structures of bones, joints, muscles and sensory and motor nerves. It is difficult to model the versatile dynamics of a leg. Standing, walking, running and manipulating objects with the foot each require the interplay of several muscle groups, including muscles outside the foot and ankle area. Moreover, the fine-tuning of foot movement and the co-ordination of different types of movement needed for foot performance is carried out in several areas of the brain. For example:
- The parietal lobe sensorimotor cortex is activated by basic, crude foot and leg function.
- The auditory and visual cortex handle audio-visual cues and adjust foot movements accordingly.
- Associative cortex aligning sensorimotor cortex provides more sophisticated analysis of foot sensing based on information from peripheral sensory nerves to the brain that is used to adjust motor nerve activities that innervate muscles that move the foot in different ways.
- Frontal lobe networks participate in planning and co-ordinating of dynamic foot function, taking into account a context of foot movement.
- Temporal lobe long-term memory and distributed memory of life events contain memories of dynamic foot skills that have been learned and will be used later.

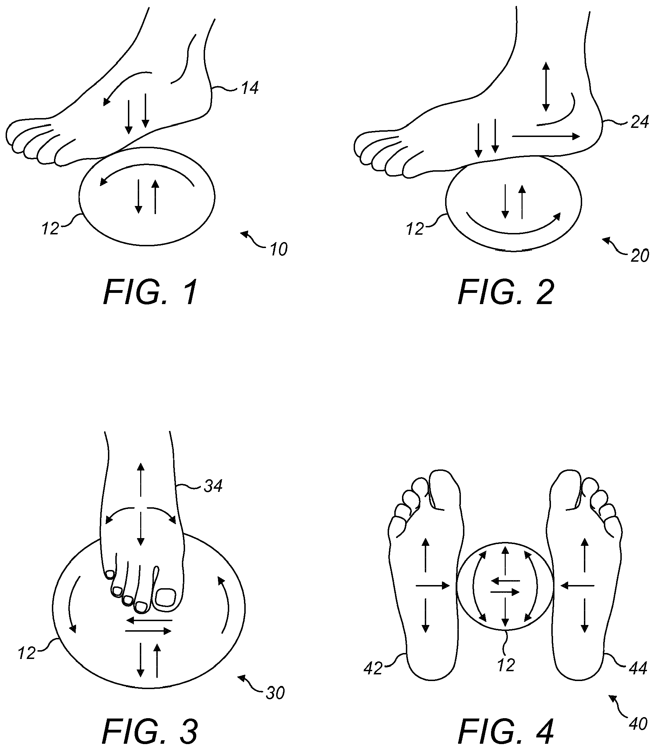

FIG. 1 is a schematic view, indicated generally by the reference numeral 10, showing an example use of a sensing ball 12 in accordance with an example embodiment. In the view 10, a foot 14 is applying a downwards force and a forward rotational force on the ball 12. The forces applied by the foot 14 and detected at the sensing ball 12 are shown by arrows in FIG. 1. As described further below, the sensing ball 12 may include sensors for obtaining data relating to the interaction of the foot 14 with the sensing ball 12.

The sensing ball 12 may be compressible such that it can react to one or more forces applied by the foot of a user. The sensing ball 12 may comprise a fluid cavity. The sensing ball 12 may have a generally spherical shape, but it is not a requirement of all embodiments that the sensing ball be a perfectly spherical. For example, the sensing ball may deviate slightly from a perfectly spherical shape. Moreover, the sensing balls maybe non-spherical, such as other shapes that can be manipulated in similar ways to balls (such as tubes). In some embodiments, the sensing balls maybe adapted such that said sensing balls do not move like balls (such as sensing balls having triangular shapes).

FIG. 2 is a schematic view, indicated generally by the reference numeral 20, showing an example use of the sensing ball 12 in accordance with an example embodiment. In the view 20, a foot 24 is applying upwards and downwards forces, in addition to rotational forces on the ball. The sensing ball 12 may include sensors for obtaining data relating to the interaction of the foot 24 with the sensing ball 12.

FIG. 3 is a schematic view, indicated generally by the reference numeral 30, showing an example use of the sensing ball 12 in accordance with an example embodiment. In the view 30, a foot 34 is applying side-to-side rotational forces and forward and backwards forces to the sensing ball. The sensing ball 12 may include sensors for obtaining data relating to the interaction of the foot 34 with the sensing ball 12.

FIG. 4 is a schematic view, indicated generally by the reference numeral 40, showing an example use of the sensing ball 12 in accordance with an example embodiment. In the view 40, a left foot 42 and a right foot 44 are collectively applying forces to the sensing ball 12. The sensing ball 12 may include sensors for obtaining data relating to the interaction of the feet 42 and 44 with the sensing ball 12.

Clearly, the movements shown in FIGS. 1 to 4 are by way of example only; many other interactions between a foot/ feet of a user and a sensing ball will be apparent to the skilled person.

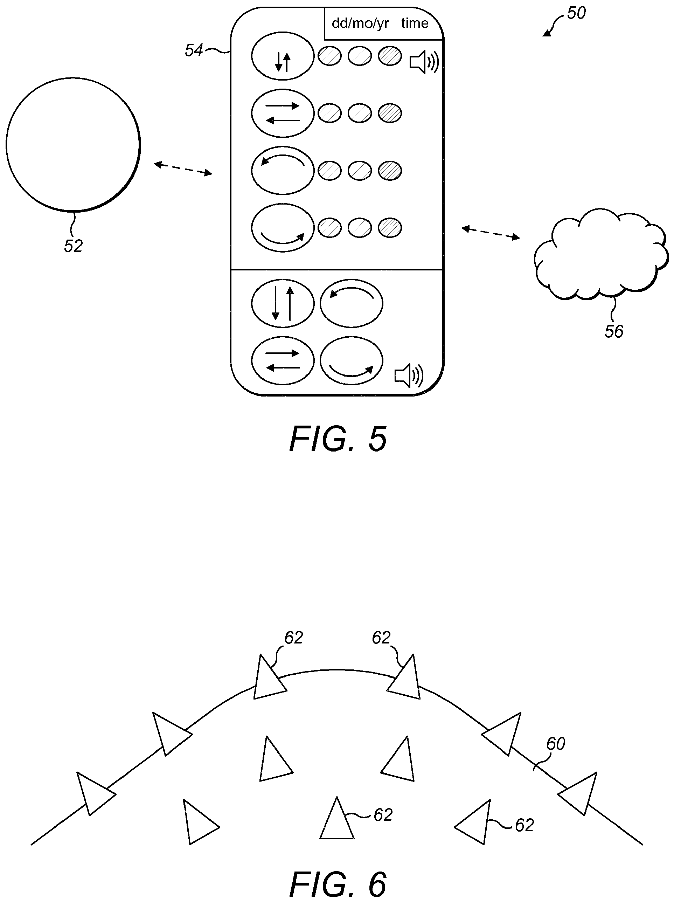

FIG. 5 is a block diagram of a system, indicated generally by the reference numeral 50, in accordance with an example embodiment. The system 50 comprises a sensing ball 52 (similar to the sensing ball 12 described above), a user interface 54 and a network or remote server 56. The network/remote server 56 may, for example, provide data storage. As shown on FIG. 5, the sensing ball 52 may be in two-way communication with the user interface 54 and the user interface 54 may be in two-way communication with the network/remote server 56. However, this arrangement is not essential. For example, one or both of those connections may provide one-way communication and one or other of those connections may be omitted in some embodiments.

The sensing ball 52 may generate data (e.g. by detecting, obtaining or otherwise receiving data) relating an interaction of a foot of a user with the sensing ball. Some or all of these data may be provided to the user interface 54; moreover, some of the data may be processed before being provided to the user interface 54. The user interface 54 may also provide instructions or information to the sensing ball 52. For example, as described further below, the sensing ball 52 may include one or more actuator means for providing a prompt to a user of the sensing ball; information regarding such prompts may be provided by the user interface 54.

The user interface 54 includes a display that can be used to provide a user with information regarding the sensing ball. The display may, for example, provide information relating to one of more of: prompts being provided to a user of the sensing ball 52; instructions to a user regarding how to interact with the sensing ball; data relating to the interaction of a foot of the user with the sensing ball; and historical information relating to user interaction with the sensing ball.

The sensing ball 12 or 52 may include one or more actuator means for providing a prompt to a user of the sensing ball. Thus, in addition to generating (e.g. detecting, obtaining or otherwise receiving) data relating an interaction of a foot of a user with the sensing ball, the sensing ball may prompt the user in some way.

FIG. 6 shows a portion of a sensing ball, indicated generally by the reference numeral 60, in accordance with an example embodiment. The sensing ball 60 includes a plurality of protrusions 62. Some or all of the protrusions 62 may be extendible and/ or retractable (for example under the control of a user interface, such as the user interface 54, or some other control module). In this way, the dimensions of the protrusions may be adjustable in a manner that can be detected by a user of the sensing ball 60, thereby providing a mechanism for providing a prompt to a user of the sensing ball 60. By way of example, a user maybe instructed to perform an action, whilst the protrusions are extending, until they can feel the protrusions in contact with the foot.

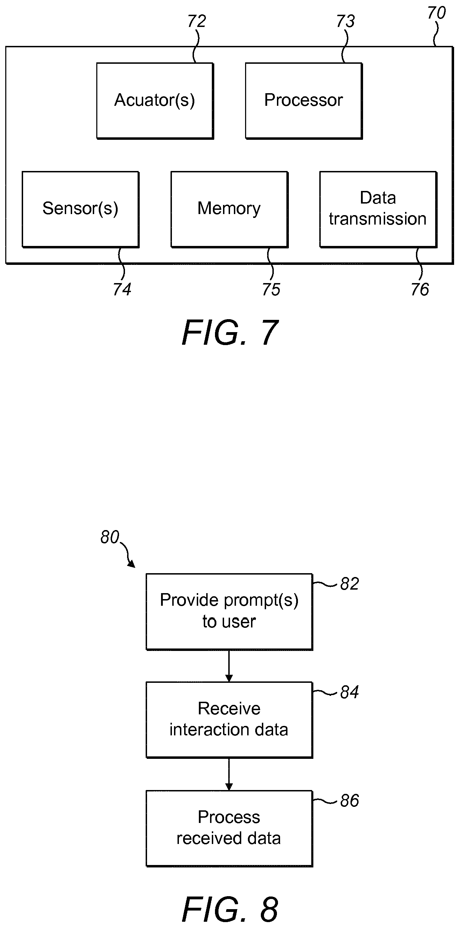

FIG. 7 is a block diagram of a system, indicated generally by the reference numeral 70, in accordance with an example embodiment. The system 70 may provide a control module, for example for a sensing ball (such as the sensing balls 12, 52 and 60 described above).

The system 70 comprises one or more actuators 72, a processor 73, one or more sensors 74, a memory module 75 (e.g. a data-logger) and a data transmission module 76. The system 70 is provided by way of example only. One or more of the elements of the system 70 may be omitted or the functionality of that element provided elsewhere in an overall system (e.g. as part of the network or remote server 56).

The one or more actuators 72 of the system 70 may be used to provide a prompt to a user of a sensing ball. For example an actuator signal (such as a vibration or a sound) may be provided by one or more of the actuators 72.

As described above, with reference to FIG. 6, the actuator(s) may comprise protrusions that may be extendible and/ or retractable; however, this is not essential to all embodiments. Other forms of actuators include: a vibration means, a haptic actuator (including, but not limited to actuators incorporating vibration means and protrusions), light and/or sound effects, and heating and/or cooling elements. Of course, alternative actuators could be provided and at least some of the actuators could be provided in combination. Thus, a wide range of actuator signals could be generated in accordance with example embodiments.

The processor 73 may be provided for processing data relating to the interaction of the foot of a user with a sensing ball. Such processing may be provided, for example, to seek to understand foot function, in which a sensing ball is used to obtain an array of information regarding foot function (and not merely a map of applied pressure). Thus, the processor 73 may gather data regarding dynamic functions of a foot (and possible other elements, such as an ankle) of a subject that are produced by different combinations of muscle, joint, sensorimotor and cognitive functions.

The processor 73 may perform one of more of gathering, storing and manipulating data related to the interaction of the foot of the user with the sensing ball. Moreover, the processor 73 may combine data relating to user interaction with a sensing ball with other metrics, such as one or more of cardio-vascular performance, breathing, eye movement, EEG data etc. The processor 73 may also comprise a timer for recording a relationship between the prompt to said user and the interaction of the foot of the user with the sensing ball.

The one or more sensors 74 of the system 70 may be used for generating, receiving (or otherwise obtaining) data relating to an interaction of a foot of the user with the sensing ball in response to the prompt. Such data may, for example, relate to changes in one or more of pressure, friction and motion induced on the sensing ball by the action of the user's foot.

Example sensors 74 include: one or more gyroscopes (for example, to measure rotation of a sensing ball); one or more accelerometers; one or more inertial magnetometers; one or more force and/or pressure sensors (for example to measure pressing by a foot of a user of a sensing ball). Of course, alternative sensors could be provided and at least some of the sensors could be provided in combination.

The memory module 75 is a means for storing data (e.g. the sensor data described above and/or data processed by the processor 73 described above). The memory module 75 may take the form of a data-logger. The memory module 75 may be provided as part of the system 70; alternatively, or in addition, data storage functionality may be provided elsewhere (e.g. as part of the network or remote server 56 described above).

The data transmission module 76 may provide a communication means with a control module. The communication means maybe a wireless communication means. The communication means may provide processed data to a control module. The communication means may receive instructions from the control module. By way of example, processed data may be provided to the network/remote server 56 by the data transmission module 76 and instructions (such as prompt instructions) may be received from the network/ remote server 56 by the data transmission module 76.

FIG. 8 is a flow chart showing an algorithm, indicated generally by the reference numeral 80, in accordance with an example embodiment.

The algorithm 80 starts at operation 82 where a prompt is provided to a user of a sensing ball (e.g. by the actuator(s) 72 of the system 70).

At operation 84, data relating to an interaction of a foot of the user with the sensing ball in response to the prompt is received (e.g. via the sensor(s) 74 of the system 70).

At operation 86, the data relating to the interaction of the foot of the user with the sensing ball is processed (e.g. by the processor 73 of the system 70).

A number of tasks can be developed and deployed in order to study aspects of foot function. Some example tasks are described further below, by way of example.

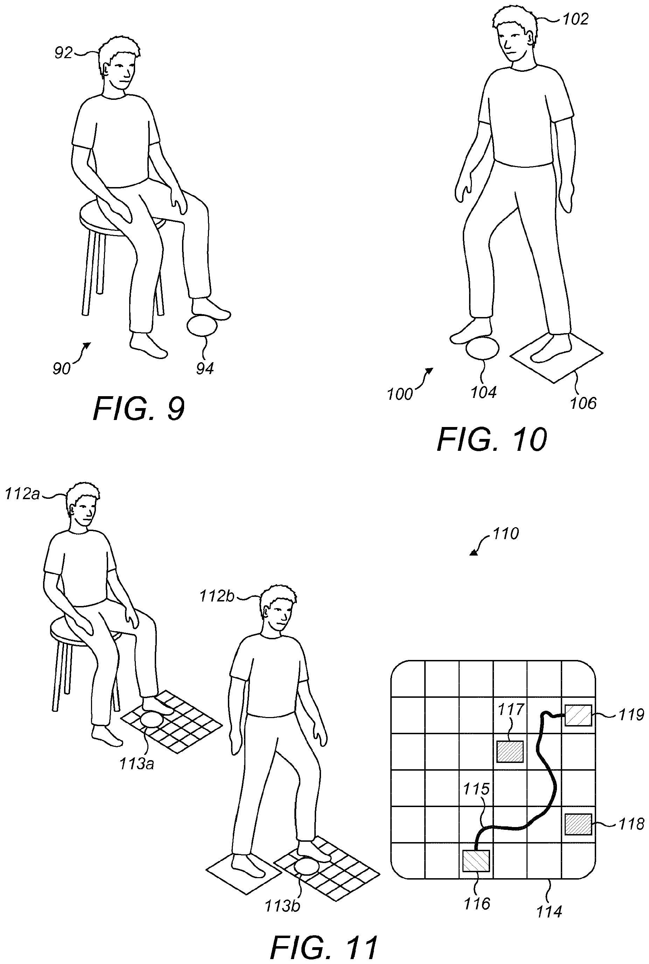

FIG. 9 is a system, indicated generally by the reference numeral 90, demonstrating an example use of an apparatus in accordance with example embodiments. In the system 90, a user 92, in a seated position, is interacting with a sensing ball 94. In the specific system 90, the user is interacting with the sensing ball 94 using a left foot. Alternatively, or in addition, the right foot or both feet could be used for interacting with the sensing ball 94.

With the user 92 in the seating position, a wide variety of tasks could be performed, with data being captured during those tasks. For example, the user may press the sensing ball 94 against the floor (or against a platform, not shown) until an actuator signal is received (e.g. a vibration is felt or a sound is heard). The vibration or sound may be provided by an actuator of a control module. The press and release of the ball may be detected using one or more sensors of the control module. The timing of the press and release of the ball may be compared with the actuator signals, for example to determine reaction times or the extent to which vibrations can be detected by the foot of the user 92.

Other tasks that might be implemented include:
- Pressing the sensing ball 94 in synchronism with an actuator signal;
- Rotating the sensing ball 94 alternatively clockwise and anti-clockwise (e.g. changing in response to an actuator signal)
- Moving the sensing ball 94 alternatively forwards and backwards (e.g. changing in response to an actuator signal).

FIG. 10 is a system, indicated generally by the reference numeral 100, demonstrating an example use of an apparatus in accordance with example embodiments. In the system 100, a user 102, in a standing position, is interacting with a sensing ball 104. In the specific system 100, the user is interacting with the sensing ball 104 using a right foot. The left foot is on a pad 106, which pad may incorporate a pressure sensor.

With the user 102 in the standing position shown in FIG. 10, a wide variety of tasks could be performed, including:
- Pressing the sensing ball 104 against the floor (or against a platform, not shown) until an actuator signal is provided/ detected (e.g. a vibration is felt or a sound is heard)
- Pressing the sensing ball 104 in synchronism with an actuator signal;
- Rotating the sensing ball 104 alternatively clockwise and anti-clockwise (e.g. changing in response to an actuator signal)
- Moving the sensing ball 104 alternatively forwards and backwards (e.g. changing in response to an actuator signal).

FIG. 11 is a system, indicated generally by the reference numeral 110, demonstrating an example use of an apparatus in accordance with example embodiments. In the system 110, a user 112 is either in a sitting position 112a and interacting with a ball 113a or a standing position 112b and interacting with a ball 113b. In the specific system 100, the user is interacting with the sensing ball 113a or 113b using a left foot.

The user 112 is presented with a user interface 114. The user 112 aims to plot a path on the user interface from a start position 116 to an end position 119, whilst avoiding positions 117 and 118. The path is indicated by the reference numeral 115. A time for completing the task may be recorded.

Clearly, although a number of tasks have been described above with reference to FIGS. 9 to 11, many other tasks may be developed and deployed in order to study aspects of foot function (or other functions, such as a combination of foot and ankle functions).

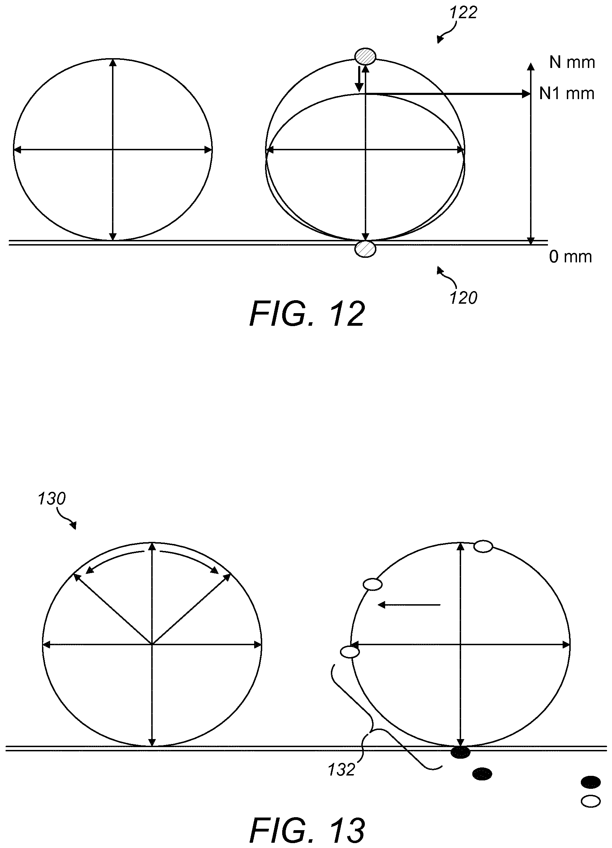

FIG. 12 shows a sensing ball, indicated generally by the reference numeral 120, demonstrating principles in accordance with example embodiments. The sensing ball 120 shows how a force applied to the sensing ball results in a compression, indicated generally by the reference numeral 122, having dimensions that can be measured (compared with an uncompressed sensing ball).

FIG. 13 shows a sensing ball, indicated generally by the reference numeral 130, demonstrating principles in accordance with example embodiments. The sensing ball 130 shows how a degree of rotation of the sensing ball can be determined by calculating an arc formed between a baseline position and a new position (as indicated generally by the reference numeral 132).

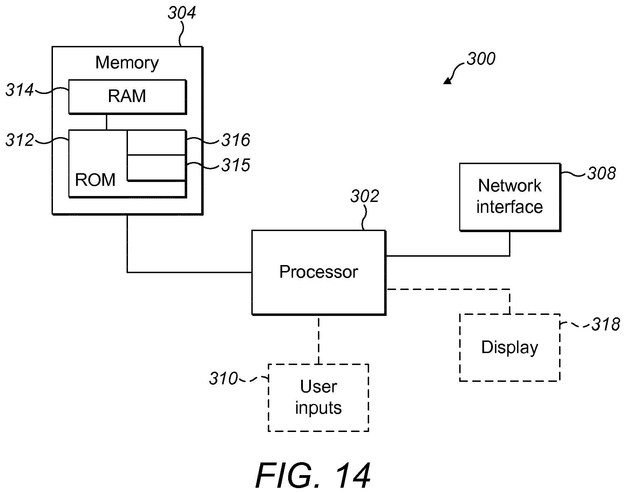

For completeness, FIG. 14 is a schematic diagram of components of one or more of the example embodiments described previously, which hereafter are referred to generically as processing systems 300. A processing system 300 may have a processor 302, a memory 304 closely coupled to the processor and comprised of a RAM 314 and ROM 312, and, optionally, user input 310 and a display 318. The processing system 300 may comprise one or more network/ apparatus interfaces 308 for connection to a network/ apparatus, e.g. a modem which maybe wired or wireless. Interface 308 may also operate as a connection to other apparatus such as device/ apparatus which is not network side apparatus. Thus direct connection between devices/ apparatus without network participation is possible.

The processor 302 is connected to each of the other components in order to control operation thereof.

The memory 304 may comprise a non-volatile memory, such as a hard disk drive (HDD) or a solid state drive (SSD). The ROM 312 of the memory 314 stores, amongst other things, an operating system 315 and may store software applications 316. The RAM 314 of the memory 304 is used by the processor 302 for the temporary storage of data. The operating system 315 may contain code which, when executed by the processor implements aspects of the algorithm 80 described above. Note that in the case of small device/ apparatus the memory can be most suitable for small size usage i.e. not always hard disk drive (HDD) or solid state drive (SSD) is used.

The processor 302 may take any suitable form. For instance, it may be a microcontroller, a plurality of microcontrollers, a processor, or a plurality of processors.

The processing system 300 may be a standalone computer, a server, a console, or a network thereof. The processing system 300 and needed structural parts may be all inside device/ apparatus such as IoT device/ apparatus i.e. embedded to very small size

In some example embodiments, the processing system 300 may also be associated with external software applications. These may be applications stored on a remote server device/ apparatus and may run partly or exclusively on the remote server device/ apparatus. These applications maybe termed cloud-hosted applications. The processing system 300 may be in communication with the remote server device/ apparatus in order to utilize the software application stored there.

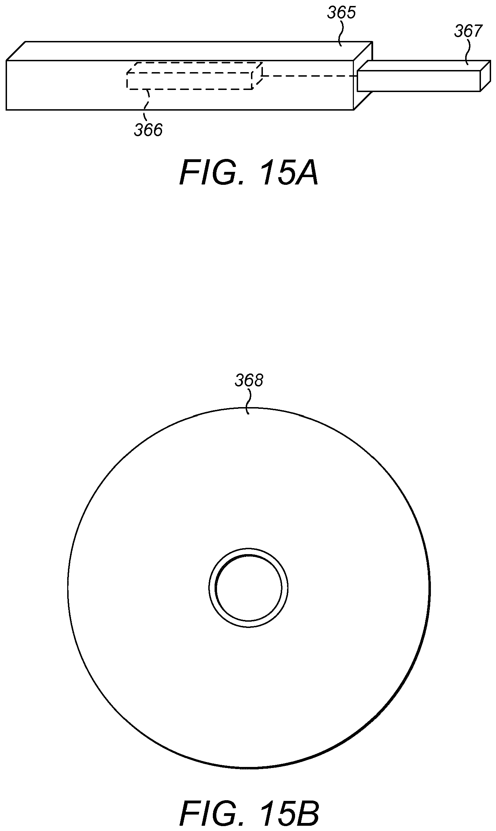

FIGS. 15A and 15B show tangible media, respectively a removable memory unit 365 and a compact disc (CD) 368, storing computer-readable code which when run by a computer may perform methods according to example embodiments described above. The removable memory unit 365 may be a memory stick, e.g. a USB memory stick, having internal memory 366 storing the computer-readable code. The memory 366 may be accessed by a computer system via a connector 367. The CD 368 may be a CD-ROM or a DVD or similar. Other forms of tangible storage media may be used. Tangible media can be any device/ apparatus capable of storing data/information which data/ information can be exchanged between devices/apparatus/network.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" may be any non-transitory media or means that can contain, store, communicate, propagate or transport the instructions for use by or in connection with an instruction execution system, apparatus, or device, such as a computer.

Reference to, where relevant, "computer-readable storage medium", "computer program product", "tangibly embodied computer program" etc., or a "processor" or "processing circuitry" etc. should be understood to encompass not only computers having differing architectures such as single/multi-processor architectures and sequencers/ parallel architectures, but also specialised circuits such as field programmable gate arrays FPGA, application specify circuits ASIC, signal processing devices/ apparatus and other devices/ apparatus. References to computer program, instructions, code etc. should be understood to express software for a programmable processor firmware such as the programmable content of a hardware device/ apparatus as instructions for a processor or configured or configuration settings for a fixed function device/ apparatus, gate array, programmable logic device/ apparatus, etc.

As used in this application, the term "circuitry" refers to all of the following: (a) hardware-only circuit implementations (such as implementations in only analogue and/or digital circuitry) and (b) to combinations of circuits and software (and/or firmware), such as (as applicable): (i) to a combination of processor(s) or (ii) to portions of processor(s)/ software (including digital signal processor(s)), software, and memory(ies) that work together to cause an apparatus, such as a server, to perform various functions) and (c) to circuits, such as a microprocessor(s) or a portion of a microprocessor(s), that require software or firmware for operation, even if the software or firmware is not physically present.

If desired, the different functions discussed herein maybe performed in a different order and/or concurrently with each other. Furthermore, if desired, one or more of the above-described functions may be optional or may be combined. Similarly, it will also be appreciated that the flow diagram of Figure 8 is an example only and that various operations depicted therein maybe omitted, reordered and/or combined.

It will be appreciated that the above described example embodiments are purely illustrative and are not limiting on the scope of the invention. Other variations and modifications will be apparent to persons skilled in the art upon reading the present specification.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

## Claims

1. An apparatus comprising:
an actuator means for providing a prompt to a user of a sensing ball;
a sensing means for generating data relating to an interaction of a foot of the user with the sensing ball in response to the prompt; and
a processing means for processing said data relating to the interaction of the foot of the user with the sensing ball.

2. An apparatus as claimed in claim 1, wherein said actuator means comprises a user interface.

3. An apparatus as claimed in claim 1 or claim 2, wherein the actuator means comprises one or more of:
a haptic actuator;
a vibration means;
one or more protrusions having controllable dimensions;
light;
sound generating means; and
heating and/or cooling means.

4. An apparatus as claimed in any one of the preceding claims, wherein the sensing means comprises a plurality of sensors.

5. An apparatus as claimed in claim 4, wherein the plurality of sensors comprises one or more of: one or more gyroscopes; one or more accelerometers; one or more inertial magnetometers; one or more force sensors; and one or more pressure sensors.

6. An apparatus as claimed in any one of the preceding claims, wherein said processing means comprises means for storing data.

7. An apparatus as claimed in any one of the preceding claims, wherein said processing means comprises a communication means for communicating with a control module.

8. An apparatus as claimed in claim 7, wherein said communication means is configured to provide processed data to the control module.

9. An apparatus as claimed in claim 8 or claim 9, wherein said communication means is configured to receive instructions from the control module.

10. An apparatus as claimed in any one of the preceding claims, wherein the processing means comprises a timer for recording a relationship between the prompt to said user and the interaction of the foot of the user with the sensing ball.

11. A sensing ball comprising an apparatus as claimed in any one of the preceding claims.

12. A sensing ball as claimed in claim 11, wherein the sensing ball is compressible.

13. A sensing ball as claimed in claim 11 or claim 12, wherein said sensing ball comprises a fluid cavity.

14. A method comprising:
providing a prompt to a user of a sensing ball;
generating data relating to an interaction of a foot of the user with the sensing ball in response to the prompt; and
processing said data relating to the interaction of the foot of the user with the sensing ball.

15. A computer readable medium comprising program instructions stored thereon for performing at least the following:
providing a prompt to a user of a sensing ball;
generating data relating to an interaction of a foot of the user with the sensing ball in response to the prompt; and
processing said data relating to the interaction of the foot of the user with the sensing ball.
